# EUROPEAN PATENT SPECIFICATION

(11) **EP 0 500 038 B1**
(45) Date of publication and mention of the grant of the patent: **25.06.1997**
(21) Application number: 92102670.4
(22) Date of filing: 18.02.1992
(51) Int. Cl.: C07C 209/84, C07B 63/00

(54) **Process for producing and purifying aliphatic amines**
Verfahren zur Produktion und Reinigung von aliphatischen Aminen
Procédé pour la production et purification d'amines aliphatiques

(30) Priority: 22.02.1991 JP 28420/91
(43) Date of publication of application: 26.08.1992
(60) Divisional application: 95113235.6
(73) Proprietor: KAO CORPORATION, Chuo-ku, Tokyo (JP)
(72) Inventor: Abe, Hiroshi, Albany, CA 94706 (JP); Taniguchi, Hideki, Naga-gun, Wakayama (JP); Tachizawa, Osamu, Wakayama-Shi, Wakayama (JP); Sotoya, Kohshiro, Naga-gun, Wakayama (JP)
(74) Representative: Hansen, Bernd, Dr. Dipl.-Chem.

(56) References cited:
- EP-A- 0 312 253
- DE-A- 1 768 743
- DE-B- 1 162 848
- GB-A- 717 680
- GB-A- 1 096 707

## Description

The present invention relates to a process for producing an aliphatic tertiary amine.

Aliphatic amines prepared from tallow, coconut oil or palm oil are important intermediates for domestic and industrial products. Particularly, aliphatic tertiary amines are used in a wide field including a softener for clothes, an antistatic agent, a gasoline additive, a shampoo, a conditioner, a microbicide and a detergent.

A process for producing such an amine from a fat and/or an oil through a fatty acid and a nitrile and a process for producing an amine from a fat and/or an oil through a higher alcohol have been known (See "Kazuhiko Okabe and Hirosi Abe, Yukagaku", Vol.37, No.7, p.487, 1988). However, aliphatic amines prepared by these conventional processes have problems such that they are colored in the conversion thereof into derivatives and also they become turbid when stored for a long time to give products which are poor in appearance.

GB-A-10 96 707 discloses a process for the purification of tertiary amines which comprises contacting the amine in the liquid phase or in solution with an activated natural earth and then separating the tertiary amine from the activated natural earth. The amine is produced by reacting three molecules of a primary alcohol with one molecule ammonia. Reference is continuously made to trilaurylamine.

DE-A-17 68 743 relates to a method for producing long chain amines by reduction of alkyl nitriles. The amines contain impurities such as amides, ketones, nitriles, paraffins, olefins and carboxylic acids. It is particularly disadvantageous that amides are produced. The amines produced are heated in the presence of alkalis and then distilled.

The object of the present invention is to provide a process for producing high-quality aliphatic tertiary amines which can be converted into useful derivatives without suffering from coloration or turbidity even after long-term storage.

The present invention provides a process for producing an aliphatic tertiary amine which comprises the steps of:
(I) reacting an aliphatic primary alcohol with dimethylamine,
(II) purifying the tertiary amine product mixture obtained with at least one step selected from (h) and (i):
   (h) contacting the reaction product mixture with an adsorbent and separating and recovering the amine from the adsorbent,
   (i) contacting the product mixture with an adsorbent in the presence of an inorganic alkali or an aqueous solution thereof and separating and recovering the amine from the adsorbent;
      and also optionally with step (j):
   (j) contacting the amine with water and separating and recovering the amine from water and/or contacting the amine with an aqueous solution of an inorganic alkali and separating and recovering the amine from the solution;
(III) and distilling the purified product to produce the aliphatic tertiary amine.

The aliphatic primary alcohol to be used in step (I) is preferably a saturated or unsaturated aliphatic primary alcohol having 8 to 22 carbon atoms and the reaction of this step is represented by the following reaction formula: 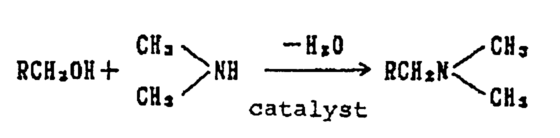 wherein R is a saturated or unsaturated aliphatic group having 7 to 21 carbon atoms.

In this step, an aliphatic primary alcohol and dimethylamine are used at a molar ratio of about 1.0 : 1.0 to 5.0 and a catalyst comprising Cu or Ni as the main component is used. Examples of the catalyst include Cu-Ni, Cu-Zn and Cu-Co and catalysts prepared by adding at least one element selected from among Pd, Pt, Ru and Rh among Group VIII platinum metals to these catalyst. The amount of the catalyst to be used is 0.1 to 5% by weight based on the aliphatic primary alcohol. The reaction temperature is generally 150 to 230°C and the pressure is from atmospheric pressure to 100 atm (gauge pressure).

The aliphatic tertiary amine (reaction product aliphatic amines, including impurities) prepared by the above step include those having 8 to 66 carbon atoms in total and particular examples thereof include dodecylamine, hexadecylamine, octadecylamine, behenylamine, didodecylamine, dihexadecylamine, dioctadecylamine, N-methyldidodecylamine, N-methyldioctadecylamine, N,N-dimethyldodecylamine, N,N-dimethylhexadecylamine, N,N-dimethyloctadecylamine, tridodecylamine, dioleylamine, trioctylamine, N-dodecyloctadecylamine and N-dodecyl-N-methyl-octadecylamine.

The reaction product prepared by step I is treated in at least one step selected from the group consisting of steps (h) and (i) and optionally (j). Steps (h) to (j) will now be described in more detail.

The adsorbent to be used in step (h) is one comprising (i) activated carbon and/or (ii) at least one member selected from the group consisting of silica, magnesia and alumina as the preferred main component(s), and examples of the adsorbent (ii) include zeolite, silica/alumina, activated alumina, Kyoward (a product of Kyowa Chemical Industry Co., Ltd.) and activated clay.

In this step, the adsorbent is added to the reaction product aliphatic amine in an amount of 0.01 to 10% by weight based on the amine and the obtained mixture is stirred at a temperature of room temperature to 150°C and filtered to separate the aliphatic amine from the adsorbent. When the amount of the adsorbent used is less than 0.01% by weight, no objective effect can be attained, while when it exceeds 10% by weight, the yield of the aliphatic amine will be low.

The step (i) is the same as above step (h) except for the presence of an inorganic alkali, so that it may be conducted under the same conditions as those of step (h) except that an inorganic alkali is additionally used. The inorganic alkali is preferably at least one member selected from the group consisting of alkali metal hydroxides, alkali metal carbonates and alkali metal hydrogencarbonates and specific examples thereof include sodium hydroxide, potassium hydroxide, sodium carbonate, potassium carbonate, sodium hydrogencarbonate and potassium hydrogencarbonate, which may be used in various forms such as powder, aqueous solution and flake.

The amount of the inorganic alkali to be used is 0.001 to 5% by weight based on the weight of the reaction product aliphatic amine.

The amount of water or aqueous solution of an inorganic alkali to be used in the step (j) is 0.01 to 20% by weight based on the weight of the reaction product aliphatic amine. The contact of the reaction product aliphatic amine with water or the aqueous solution of an inorganic alkali is conducted while stirring at room temperature to 150°C for 0.1 to 10 hours. The inorganic alkali to be used in this step may be one selected from among those materials usable in step (i). The concentration of the aqueous solution is preferably 0.1 to 50% by weight.

According to the present invention, one step among steps (h)and (i) may be conducted, or the steps (h) and (i) may be repeated an arbitrary number of times and the order of repetition of these steps is not restricted. For example, only step (h) may be conducted several times; or the optional step (j) may be conducted, before the step (h); or step (i) may be conducted several times before step (h). Alternatively, steps (h) and (j) may be conducted simultaneously or steps (h) and (i) may be conducted simultaneously.

The process of the present invention provides a high-quality aliphatic amine which can be converted into a derivative without suffering from coloration and does not become turbid even after long-term storage.

### Example 1 and Comparative Example 1

600 g of dodecyl alcohol (a product of Kao Corporation; Kalcohl 20) and 3 g (corresponding to 0.5% by weight based on the alcohol used as the raw material) of a copper-nickel catalyst were fed into a 1-ℓ four-necked flask. The system was purged with nitrogen while stirring and the temperature rise of the system was initiated. When the temperature of the system reached 100°C, hydrogen gas was blown into the system at a flow rate of 40 ℓ/hr with a flowmeter and the temperature of the system was raised to a reaction temperature, i.e., 200°C. At this temperature, the introduction of dimethylamine gas was initiated and a reaction was conducted for 5 hours. After the completion of the reaction, the reaction mixture was filtered to remove the catalyst, thereby providing crude N,N-dimethyldodecylamine. The crude N,N-dimethyldodecylamine was put in a 1-ℓ four-necked flask, followed by the addition of 3 g of activated carbon and 3 g of Kyoward 600 S (a product of Kyowa Chemical Industry Co., Ltd., main components: silica 64.9%, magnesia 13.5%, both listed values). The contents were stirred at 90°C in a nitrogen atmosphere for about 2 hours and filtered to remove the adsorbent. The filtrate was purified by distillation to give N,N-dimethyldodecylamine. The distillation was conducted at 5 Torr.

The amine prepared above (Example 1) and the amine (Comparative Example 1) prepared by the same procedure as that described above except that no treatment with activated carbon and Kyoward 600S was conducted prior to the distillation, were examined for quality just after preparation and after storage in air at 60°C for one month by the following method and the results are given in Table 1.

### 〈Test method of quality〉

A solution prepared by mixing 20 g of an amine sample with 20 g of ethanol and 10 g of 35% hydrochloric acid is kept at 60°C for 15 minutes to determine the color change of the solution with a Lovibond colorimeter (using a 5 1/4 inch cell).

**Table 1**

| | Lovibond Red | |
|---|---|---|
| | Example 1 | Comparative Example 1 |
| treatment with activated carbon and Kyoward | made | not made |
| just after preparation | 0.1 or below | 0.1 or below |
| after the storage for one month | 0.1 or below | 22.7 |

It can be understood from the above results that a high-quality amine can be prepared by treating a crude amine with activated carbon and Kyoward.

### Examples 2, 3 and 4 and Comparative Example 2

Crude N,N-dimethylstearylamine was prepared in a similar manner to that of Example 1 except that stearyl alcohol (a product of Kao Corporation, Kalcohl 80) was used instead of the dodecyl alcohol. The crude N,N-dimethylstearylamine was treated by various methods specified in Table 2 and purified by distillation. The obtained amines were stored in the air at 30°C for one month to examine the appearance (whether they were turbid or clear).

The results are given in Table 2.

**Table 2**

| | Method for treating crude amine | Appearance after one month |
|---|---|---|
| Ex. 2 | 48% aqueous solution of NaOH, activated carbon and Kyoward 600S were added to crude amine (each in an amount 0.5% by weight based on the crude amine) and the obtained mixture was stirred at 90°C for 2 hours in a nitrogen atmosphere and filtered to remove the adsorbents. The obtained amine was purified by distillation and stored for one month. | clear |
| Ex. 3 | activated carbon and Kyoward 600S were added to crude amine (each in an amount of 0.5% by weight based on the crude amine) and the obtained mixture was stirred at 90°C for 2 hours in a nitrogen atmosphere and filtered to remove the adsorbents. 1% (based on the amine) of water was added to the filtrate and the obtained mixture was maintained at 50°C for about one hour, distilled and stored for one month. | clear |
| Ex. 4 | activated carbon and Kyoward 600S were added to crude amine (each in an amount of 0.5% by weight based on the crude amine) and the obtained mixture was stirred at 90°C for 2 hours in a nitrogen atmosphere and filtered to remove the adsorbents. the filtrate was distilled, followed by the addition of 300 ppm (based on the amine) of tocopherol and the obtained mixture was stored for one month. | clear |
| Comp. Ex. 2 | no treatment was conducted to crude amine except for distillation and the obtained distillate was stored for one month. | turbid |

It can be understood from the above results that an amine which does not become turbid even after storage for one month can be obtained by treating a crude amine by at least one step selected from among above steps (h) and (i) and optionally step (j).

## Claims

1. A process for producing an aliphatic tertiary amine which comprises the steps of:
(I) reacting an aliphatic, primary alcohol with dimethylamine,
(II) purifying the obtained tertiary amine product mixture with at least one step selected from (h) and (i):
(h) contacting the product mixture with an adsorbent and separating and recovering the amine from the adsorbent,
(i) contacting the product mixture with an adsorbent in the presence of an inorganic alkali or an aqueous solution thereof and separating and recovering the amine from the adsorbent;
and also optionally with step (j):
(j) contacting the amine with water and separating and recovering the amine from water and/or contacting the amine with an aqueous solution of an inorganic alkali and separating and recovering the amine from the solution;
(III) and distilling the purified product to produce the aliphatic, tertiary amine.

2. The process for producing an aliphatic tertiary amine according to claim 1 wherein the adsorbent used is one comprising (i) activated carbon and/or (ii) at least one member selected from the group consisting of silica, magnesia and alumina as a main component.

3. The process for producing an aliphatic tertiary amine according to claim 1 or 2 wherein the inorganic alkali used is at least one compound selected from the group consisting of an alkali metal hydroxide, an alkali metal carbonate and an alkali metal hydrogencarbonate.

4. The process for producing an aliphatic tertiary amine according to claims 1 or 2 wherein step II is conducted in an inert atmosphere.

5. The process for producing an aliphatic tertiary amine according to claims 1 or 2 wherein the aliphatic amine has a saturated or unsaturated aliphatic group having 8 to 22 carbon atoms.

6. The process for producing an aliphatic tertiary amine according to claims 1 or 2 wherein the adsorbent used is a combination of (i) activated carbon and (ii) at least one member selected from the group consisting of silica, magnesia and alumina as a main component.

7. The process for producing an aliphatic tertiary amine according to claims 1 or 2 wherein the amount of the adsorbent used is in a range of from 0.01 to 10% by weight based on the amine.

## Patentansprüche

1. Verfahren zur Erzeugung eines aliphatischen, tertiären Amins, umfassend die folgenden Schritte:
(I) Reaktion eines aliphatischen, primären Alkohols mit Dimethylamin,
(II) Reinigung der erhaltenen tertiären Amin-Produktmischung mit zumindest einem Schritt, ausgewählt aus (h) und (i):
(h) Kontaktieren der Produktmischung mit einem Adsorbens und Trennen und Wiedergewinnen des Amins von dem Adsorbens,
(i) Kontaktieren der Produktmischung mit einem Adsorbens in der Gegenwart eines anorganischen Alkalis oder einer wäßrigen Lösung davon und Trennen und Wiedergewinnen des Amins von dem Adsorbens;
und wahlweise ebenfalls mit Schritt (j):
(j) Kontaktieren des Amins mit Wasser und Trennen und Wiedergewinnen des Amins von Wasser und/oder Kontaktieren des Amins mit einer wäßrigen Lösung aus einem anorganischen Alkali und Trennen und Wiedergewinnen des Amins von der Lösung;
(III) und Destillieren des gereinigten Produktes, zur Erzeugung des aliphatischen, tertiären Amins.

2. Verfahren zur Erzeugung eines aliphatischen, tertiären Amins nach Anspruch 1, worin das verwendete Adsorbens eines ist, umfassend (i) Aktivkohle und/oder (ii) zumindest eine Verbindung, ausgewählt aus der Gruppe, bestehend aus Silica, Magnesia und Alumina als Hauptkomponente.

3. Verfahren zur Erzeugung eines aliphatischen, tertiären Amins nach Anspruch 1 oder 2, worin das verwendete anorganische Alkali zumindest eine Verbindung ist, ausgewählt aus der Gruppe, bestehend aus einem Alkalimetallhydroxid, einem Alkalimetallcarbonat und einem Alkalimetallhydrogencarbonat.

4. Verfahren zur Erzeugung eines aliphatischen, tertiären Amins nach Anspruch 1 oder 2, worin der Schritt II in einer Inertatmosphäre durchgeführt wird.

5. Verfahren zur Erzeugung eines aliphatischen, tertiären Amins nach Anspruch 1 oder 2, worin das aliphatische Amin eine gesättigte oder ungesättigte, aliphatische Gruppe mit 8 bis 22 Kohlenstoffatomen hat.

6. Verfahren zur Erzeugung eines aliphatischen, tertiären Amins nach Anspruch 1 oder 2, worin das verwendete Adsorbens eine Kombination aus (i) Aktivkohle und (ii) zumindest einer Verbindung ist, ausgewählt aus der Gruppe, bestehend aus Silica, Magnesia und Alumina als Hauptkomponente.

7. Verfahren zur Erzeugung eines aliphatischen, tertiären Amins nach Anspruch 1 oder 2, worin die Menge des verwendeten Adsorbens in einem Bereich von 0,01 bis 10 Gew.-% liegt, bezogen auf das Amin.

## Revendications

1. Procédé de production d'une amine aliphatique tertiaire qui comprend les étapes consistant à :
(I) faire réagir un alcool aliphatique primaire avec de la diméthylamine,
(II) purifier le mélange de produits d'amine tertiaire obtenu par au moins une étape choisie parmi les étapes (h) et (i) consistant à :
(h) mettre en contact le mélange de produits avec un agent adsorbant et séparer et récupérer l'amine de l'agent adsorbant,
(i) mettre en contact le mélange de produits avec un agent adsorbant en présence d'une base inorganique ou d'une solution aqueuse de celle-ci et séparer et récupérer l'amine de l'agent adsorbant; et également facultativement par l'étape (j) consistant à :
(j) mettre en contact l'amine avec de l'eau et séparer et récupérer l'amine de l'eau et/ou mettre en contact l'amine avec une solution aqueuse d'une base inorganique et séparer et récupérer l'amine de la solution;
(III) et distiller le produit purifié pour produire l'amine aliphatique tertiaire.

2. Procédé de production d'une amine aliphatique tertiaire selon la revendication 1, dans lequel l'agent adsorbant utilisé est un agent adsorbant comprenant (i) du charbon actif et/ou (ii) au moins un élément choisi parmi la silice, la magnésie et l'alumine comme composant principal.

3. Procédé de production d'une amine aliphatique tertiaire selon la revendication 1 ou 2, dans lequel la base inorganique utilisée est au moins un composé choisi parmi un hydroxyde de métal alcalin, un carbonate de métal alcalin et un hydrogénocarbonate de métal alcalin.

4. Procédé de production d'une amine aliphatique tertiaire selon la revendication 1 ou 2, dans lequel on effectue l'étape II dans une atmosphère inerte.

5. Procédé de production d'une amine aliphatique tertiaire selon la revendication 1 ou 2, dans lequel l'amine aliphatique a un groupe aliphatique saturé ou insaturé comportant 8 à 22 atomes de carbone.

6. Procédé de production d'une amine aliphatique tertiaire selon la revendication 1 ou 2, dans lequel l'agent adsorbant utilisé est une combinaison de (i) charbon actif et (ii) au moins un élément choisi parmi la silice, la magnésie et l'alumine comme composant principal.

7. Procédé de production d'une amine aliphatique tertiaire selon la revendication 1 ou 2, dans lequel la quantité de l'agent adsorbant utilisé est comprise entre 0,01 et 10 % en poids, par rapport à l'amine.
